# EUROPEAN PATENT SPECIFICATION

(11) **EP 0 397 357 B1**
(45) Date of publication and mention of the grant of the patent: **09.06.1993**
(21) Application number: 90304585.4
(22) Date of filing: 26.04.1990
(51) Int. Cl.: A61M 25/00

(54) **Monorail catheter with guidewire port marker**
Katheter mit Markierung der Öffnung für den Führungsdraht
Cathéter avec marquage de l'orifice pour le fil de guidage

(30) Priority: 08.05.1989 US 348903
(43) Date of publication of application: 14.11.1990
(73) Proprietor: SCHNEIDER (USA) INC., Plymouth, Minnesota 55442 (US)
(72) Inventor: Shockey, Rick L., Eagan, Minnesota (US); Vance, Jeffrey D., Hugo, Minnesota (US)
(74) Representative: Bradbrook, Geoffrey William

(56) References cited:
- EP-A- 0 132 215
- US-A- 3 605 750
- US-A- 4 762 129
- US-A- 4 824 435

## Description

### MONORAIL CATHETER WITH GUIDEWIRE PORT MARKER

This invention relates generally to apparatus for performing angioplasty and angiography procedures and more particularly to an improved catheter having means for identifying the location of the guidewire eye or port on a Monorail style catheter.

In performing angiographic studies and balloon angioplasty procedures, it is common practice to make a surgical incision through the skin and into an appropriate blood vessel followed with the placement of introducer. The introducer includes a suitable gasket for stemming the flow of blood when various working devices are inserted through the introducer into the vascular system. Typically, a guide catheter will be first inserted and the distal end thereof is advanced until it is sealed into the coronary ostia. Next, a guidewire is fed through the guide catheter and is advanced until it is at or slightly beyond the site where obstruction or treatment is to take place. This provides a tubular channel and track for introduction of one or more styles of working catheters.

One style of a working catheter employed generally comprises an elongated flexible plastic tubular member having a lumen extending between its distal end and its proximal end and an inflatable balloon at its distal end. In an over-the-wire system, this balloon catheter is fitted over the guidewire and advanced, with the result that the guidewire is totally contained within the lumen of the balloon catheter. More recently, applicants' assignee devised an improved balloon catheter in which a special lumen only a few centimeters long and located in the distal end portion of the catheter is provided and a port or eye is formed, either through the wall of the catheter or at the proximal end opening of the lumen to provide access to that short lumen. After the guidewire has been advanced through the vascular system in the manner indicated above, the distal end opening of the balloon catheter is fitted over the guidewire and then the guidewire is allowed to exit the balloon catheter through the guidewire eye or port. By restraining movement of the guidewire as the balloon catheter is advanced, it will ride along the guidewire with the guidewire being external to the lumen of the balloon catheter over the substantial majority of its length. Because of the manner in which the balloon catheter traverses the guidewire, it is figuratively referred to as a Monorail catheter. Further information concerning the constructional features and operation of a Monorail catheter can be obtained from the Bonzel Patent 4,762,129.

Because only a few centimeters of the lumen of the Monorail catheter engage the guidewire, care must be exercised by the surgeon when manipulating the guidewire and catheter to prevent the distal end of the guidewire from being pulled back out through the guidewire eye. Should this happen with a Monorail catheter, it would be necessary to withdraw the guide catheter, assemble it onto the proximal end of the guidewire and again advance it through the vascular system to the treatment site and thereby prolong the catheterization procedure and further damage the endothelial lining of the blood vessel.

It is accordingly a principal object of the present invention to provide an improved Monorail type balloon or working catheter.

Another object of the invention is to provide a Monorail balloon or working catheter having means for identifying the location of the guidewire eye thereon.

Yet another object of the invention is to provide a radiopaque marker on a Monorail-type guide catheter or working catheter to identify the location of the guidewire eye.

In accordance with the invention there is provided an intravascular catheter of the type comprising an elongated, flexible, plastic, tubular member having a proximal end, a distal end and at least one lumen extending between said proximal end and said distal end. A guidewire eye is located a predetermined distance proximal of the distal end of the catheter and is adapted to receive a guidewire therethrough. To avoid inadvertent uncoupling of the catheter from its guidewire, the guidewire eye is identified with a radiopaque marker so that the surgeon can perceive the location of the distal end of the guidewire relative to the catheter's guidewire eye on the screen of a fluoroscope, thus precisely displaying the point at which the catheter will uncouple from its guidewire.

The foregoing features and advantages of the invention will become apparent to those skilled in the art from the following detailed description of a preferred embodiment, especially when considered in conjunction with the accompanying drawings in which like numerals in the several views refer to corresponding parts.
Figure 1 is a side elevation view of a Monorail catheter in accordance with the present invention;
Figure 2 is a greatly enlarged cross-sectional view taken along the line 2-2 in Figure 1; and
Figure 3 is a greatly enlarged cross-sectional view taken along the line 3-3 in Figure 1.

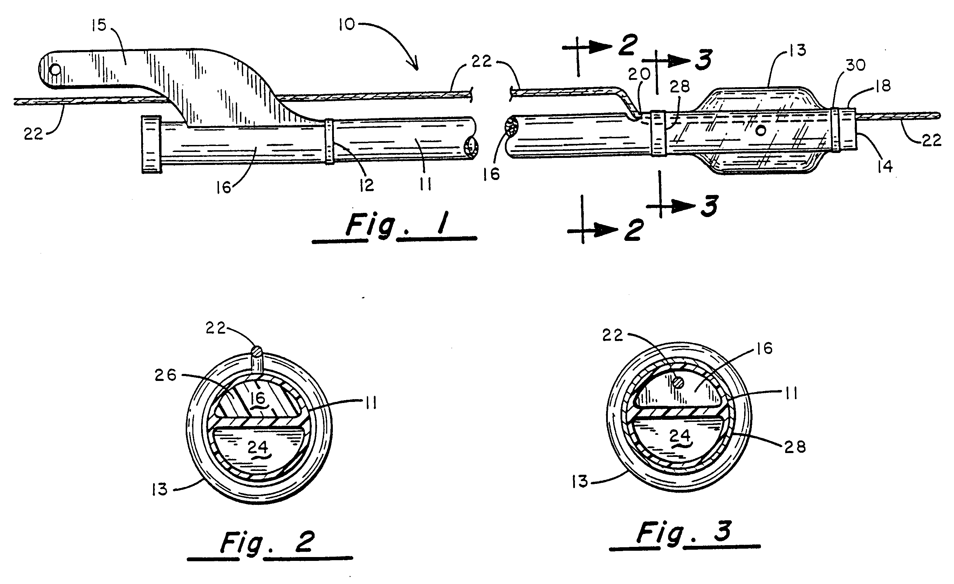

Referring first to Figure 1, there is indicated generally by numeral 10 a working catheter constructed in accordance with the present invention. It may be an angioplasty catheter in which case it includes an expander member (balloon) 13 or it may be an angiographic catheter in which case the balloon is omitted. The catheter has a proximal end 12 and a distal end 14 and at least one lumen 16 extending the entire length thereof from the proximal end to the distal end. Secured to the proximal end of the catheter body 11 is a hub member 16 which may be configured as illustrated in EP-A-0335581 and entitled "CATHETER Y-CONNECTOR WITH GUIDEWIRE LOCKING MEANS".

The tubular body 11 may also be provided with a soft, deformable, a traumatic tip 18 which typically may be formed in accordance with the method described in U.S. Patent 4,551,292, assigned to applicants' assignee.

Being a Monorail catheter, there is formed approximately 6 to 10 cms. proximal of the distal tip 18 a guidewire eye or port 20 which leads to a tubular lumen 10 within the wall of the tubular body 11. For purposes of illustration, there is shown a guidewire 22 whose distal end portion is fitted through the guidewire eye 20 and which extends through the lumen 16 and out the distal end thereof.

Referring to Figure 2, in accordance with a further feature of the invention, the catheter body 11 may have two lumens, i.e., lumen 16 and lumen 24. Lumen 16 is sealed off just proximal of the guidewire port 20, thus forming a blockage or occlusion 26 which aides in causing a guidewire threaded through the distal end of the lumen 16 to exit the guidewire port 20.

Referring next to Figure 3, there is shown a greatly enlarged cross-sectional view taken along the line 3-3 in Figure 1. This is located immediately distal of the guidewire eye or port 20 and reveals that the lumen 16 is open all the way to the distal end 14 thereof and that the guidewire 22 passes through this lumen. The lumen 24 is reserved for passage of a working fluid, e.g., an angiographic dye or an angioplasty catheter inflation fluid.

With continued reference to Figure 3, it can be seen that surrounding the tubular body 11 at a location just distal of the port 20 is a marker band 28 which typically may comprise a band of gold or other radiopaque material which will readily show up on a fluoroscopic display. While it has been common practice to include a radiopaque marker band at the distal end of a catheter as at 30 in Figure 1, by providing the band 28 at the location of the guidewire eye or port 20, it provides a means whereby the surgeon in manipulating the catheter 10 or the guidewire 22 during the course of a catheterization procedure, will be able to see when the distal end of the radiopaque guidewire 22 is approaching the port 20 to the point where increased care must be exercised to avoid the inadvertent extraction of the guidewire 22 from the guidewire lumen 16.

While the present invention has been specifically illustrated in connection with a balloon catheter, those skilled in the art will appreciate that the use of a marker band at the guidewire eye or port of any Monorail-type dilation, atherectomy, angiographic or oblation catheter may be used as well. In such devices, if the guidewire is pulled back past the entry port, the guidewire then floats free from the catheter, making it necessary to remove the catheter and subsequent re-entry and positioning of the guidewire in the catheter or blood vessel involved. The marker arrangement of the present invention allows the surgeon to locate the guidewire's distal end relative to the catheter's guidewire port.

This invention has been described herein in considerable detail in order to comply with the Patent Statutes and to provide those skilled in the art with the information needed to apply the novel principles and to construct and use such specialized components as are required. However, it is to be understood that the invention can be carried out by specifically different equipment and devices, and that various modifications, both as to the equipment details and operating procedures, can be accomplished without departing from the scope of the invention itself.

## Claims

1. An intravascular catheter (10) of the type comprising an elongated, flexible, plastic, tubular member (11) having a proximal end (12), a distal end (14) and at least one lumen (16) extending between said proximal end (12) and said distal end (14) with a guidewire eye (20) located a predetermined distance proximal of said distal end (14) and adapted to receive a guidewire (22) therethrough, characterised by :
a radiopaque marker (28) identifying the location of said guidewire eye (20) on said tubular member (11).

2. The intravascular catheter (10) as in Claim 1 wherein said radiopaque marker (28) comprises a metal band surrounding said tubular member (11) at said guidewire eye (20).

3. An intravascular catheter (10) of the type comprising a first elongated, flexible, plastic tubular member (11) having a proximal end (12), a distal end (14), a first and second lumen (16, 24) extending from said proximal end (12) to said distal end (14) a guidewire lumen (16) located in said first tubular member a short distance proximal of said distal end, characterised by :
radiopaque marker means (28) identifying the location of the proximal end of said guidewire lumen (16).

4. The intravascular catheter (10) as in Claim 3 wherein said radiopaque marker means (28) comprises a band of metal selected from the group including gold, tantalum, tungsten and titanium, said band surrounding said tubular member (11) at said guidewire eye (20).

## Patentansprüche

1. Intravaskulärer Katheter (10) der Art mit einem langgestreckten, flexiblen, rohrförmigen Glied (11) aus Kunststoff, das ein proximales Ende (12), ein distales Ende (14) und wenigstens eine Bohrung (16), die zwischen dem proximalen Ende (12) und dem distalen Ende (14) verläuft, aufweist, wobei sich in einem gegebenen bestand proximal vom distalen Ende (14) eine Führungsdraht-Durchgangsöffnung (20) befindet, durch die ein Führungsdraht (22) verlaufen kann, gekennzeichnet durch:
eine Röntgen-Sichtmarkierung (28), die die Position der Führungsdraht-Durchgangsöffnung (20) am rohrförmigen Glied (10) identifiziert.

2. Intravaskulärer Katheter (10) nach Anspruch 1, bei dem die Röntgen-Sichtmarkierung (28) ein Metallband umfaßt, das das rohrförmige Glied (11) an dieser Führungsdraht-Durchgangsöffnung (20) umgibt.

3. Intravaskulärer Katheter (10) der Art mit einem langgestreckten, flexiblen, rohrförmigen Glied (11) aus Kunststoff, das ein proximales Ende (12), ein distales Ende (14) und eine erste und eine zweite Bohrung (16, 24), die zwischen dem proximalen Ende (12) und dem distalen Ende (14) verlaufen, aufweist, wobei über eine kurze Strecke proximal vom distalen Ende eine Führungsdraht-Bohrung (16) verläuft, gekennzeichnet durch:
eine Röntgen-Sichtmarkierung (28), die die Position des proximalen Endes der Führungsdraht-Bohrung (16) identifiziert.

4. Intravaskulärer Katheter (10) nach Anspruch 3, bei dem die Röntgen-Sichtmarkierung (28) ein Metallband aus Gold, Tantal, Wolfram oder Titan umfaßt, das das rohrförmige Glied (11) an einer Führungsdraht-Durchgangsöffnung (20) umgibt.

## Revendications

1. Cathéter intravasculaire (10) du type comportant un élément tubulaire flexible et allongé (11) en matière plastique ayant une extrémité proximale (12), une extrémité distale (14) et au moins une lumière (16) s'étendant entre ladite extrémité proximale (12) et ladite extrémité distale (14), un oeil (20) pour fil de guidage étant situé à une distance prédéterminée, du côté proximal, de ladite extrémité distale (14) et étant destiné à recevoir à travers lui un fil de guidage (22), caractérisé par :
un marqueur radio-opaque (28) identifiant la position dudit oeil pour fil de guidage sur ledit élément tubulaire (11).

2. Cathéter intravasculaire (10) selon la revendication 1, dans lequel ledit marqueur radio-opaque (28) comprend un collier métallique entourant ledit élément tubulaire (11) audit oeil (20) pour fil de guidage.

3. Cathéter intravasculaire (10) du type comportant un premier élément tubulaire flexible et allongé (11) en matière plastique ayant une extrémité proximale (12), une extrémité distale (14), des première et seconde lumières (16, 24) s'étendant de ladite extrémité proximale (12) jusqu'à ladite extrémité distale (14), une lumière (16) pour fil de guidage située dans ledit premier élément tubulaire à peu de distance, du côté proximal, de ladite extrémité distale, caractérisé par :
un moyen marqueur radio-opaque (28) identifiant la position de l'extrémité proximale de ladite lumière (16) pour fil de guidage.

4. Cathéter intravasculaire (10) selon la revendication 3, dans lequel ledit moyen marqueur radio-opaque (28) comprend un collier d'un métal choisi dans le groupe comprenant de l'or, du tantal, du tungstène et du titane, ledit collier entourant ledit élément tubulaire (11) audit oeil (20) pour fil de guidage.
